# NEUE EUROPÄISCHE PATENTSCHRIFT

(11) **EP 2 608 798 B2**
(45) Veröffentlichungstag und Bekanntmachung des Hinweises auf die Entscheidung über den Einspruch: **07.11.2018**
(45) Hinweis auf die Patenterteilung: 04.03.2015
(21) Anmeldenummer: 11749166.2
(22) Anmeldetag: 26.08.2011
(51) Int. Cl.: A61K 36/53, A61P 25/28

(54) **PFLANZENEXTRAKTE AUS GLIEDKRÄUTERN UND DEREN VERWENDUNG ZUR STEIGERUNG KOGNITIVER LEISTUNGSFÄHIGKEIT**
PLANT EXTRACTS MADE OF SIDERITIS AND USE THEREOF TO BOOST COGNITIVE PERFORMANCE
EXTRAITS VÉGÉTAUX DE SIDERITIS ET LEUR UTILISATION POUR AUGMENTER LES PERFORMANCES COGNITIVES

(30) Priorität: 27.08.2010 EP 10174411
(43) Veröffentlichungstag der Anmeldung: 03.07.2013
(73) Patentinhaber: Finzelberg GmbH & Co. KG, 56626 Andernach (DE)
(72) Erfinder: FEISTEL, Björn, 56626 Andernach (DE); WALBROEL, Bernd, 53639 Königswinter (DE)
(74) Vertreter: dompatent von Kreisler Selting Werner - Partnerschaft von Patent- und Rechtsanwälten mbB
(86) Internationale Anmeldenummer: PCT/EP2011/064687
(87) Internationale Veröffentlichungsnummer: WO 2012/025609

(56) Entgegenhaltungen:
- EP-A1- 2 229 950
- WO-A2-2011/076867
- RAINER KNÖRLE ET AL: "Extrakte aus Sideritis ssp. (griechischer Bergtee): Innovative zentral aktive Pflanzenextrakte mit breitem Wirkprofil", INTERNET CITATION, 1. Oktober 2009 (2009-10-01), Seiten 1-15, XP002591208, Gefunden im Internet: URL:http://ibam.de/pics/Poster-Wolnzach-20 09.pdf [gefunden am 2010-07-08]

## Beschreibung

Die **Gliedkräuter** *(Sideritis L.)* sind eine Pflanzengattung aus der Familie der Lippenblütler (Lamiaceae). Zu dieser Gattung gehören etwa 140 Arten, welche in rund 320 Unterarten, Ökotypen und Kultivare untergliedert werden können. Die Gliedkräuter sind einjährige bis ausdauernde, krautige Pflanzen oder kleine Sträucher. Mehrere Arten werden als Teedrogen genutzt und sind als Griechischer Bergtee im Handel. Das Areal der Gattung reicht von den atlantischen Inseln West-Europas und Nordwest-Afrikas (Makronesien) über das Mittelmeergebiet bis Russland, Tibet und West-China. Das Mannigfaltigkeitszentrum befindet sich im Westen des Areals [Ramón Morales: Sideritis L., In: Flora Iberica, Band 12].

Beim Bergtee handelt es sich meist um Sideritis-Arten, deren botanische Zuordnung manchmal schwierig ist. Sie haben je nach Region unterschiedlichste Namen und zum Teil nur lokale Bedeutung. Alleine in der Türkei gibt es 46 und in Spanien 45 Arten Sideritis, die vielfach endemisch sind. Neben ihrer Artenvielfalt ist allen gemeinsam, dass sie zur Familie der Lippenblütler (*Lamiaceae*) gehören und leicht mit Salbei verwechselt werden können. In vielen Fällen pflückt und trocknet die Landbevölkerung Sideritis-Pflanzen an den Berghängen zum Eigengebrauch. Der aromatische Tee ist dann oft aus mehreren Sideritis-Species zusammengesetzt. Er wird mit kochendem Wasser aufgebrüht, entweder heiß oder kalt getrunken und kann mit Zucker oder Honig gesüßt werden. Die Verwendung als Gebrauchstee gehört in vielen Mittelmeerländern zum alltäglichen Leben, der gesundheitsfördernde Effekt ist eher nebensächlich. Trotzdem sind die medizinalen Effekte seit langem bekannt und wurden schon vor zweitausend Jahren von dem berühmten griechischen Arzt Dioskurides (1. Jahrhundert nach Christus) beschrieben. In seiner Arzneimittellehre ist beispielsweise zu lesen, dass Sideritis- Pflanzen als Umschlag angewandt «die Kraft haben, Wunden zu verkleben und Entzündungen abzuhalten». Die traditionelle Volksmedizin in den Ländern rund um das Mittelmeer hat sich seit Dioskurides weiterentwickelt. Heute verwendet man Sideritis-Pflanzen meistens als Tees - seltener als ätherische Öle. Ihr Einsatzgebiet ist sehr vielfältig: Zur Entkrampfung bei Unpässlichkeit des Magen-Darm-Trakts oder zur Beruhigung bei Schlaf- und Ruhelosigkeit. Moderne Pflanzenheilkundige nutzen die antientzündliche und antibakterielle Wirkung zur unterstützenden Behandlung von Infektionen der ableitenden Harnwege oder Blase. Kalte Tees können auch zum Gurgeln verwendet werden, um Entzündungen im Mund (Rachen und Zahnfleisch) zu lindern und schneller abheilen zu lassen. Und heiß getrunken dienen die Tees traditionell zur Vorbeugung von Erkältungskrankheiten oder insbesondere zur Bekämpfung von Atemwegserkrankungen. Wie viele andere Lippenblütler aus der Mittelmeerregion (Salbei, Thymian) sind auch die Sideritis-Arten reich an ätherischen Ölen, aber frei von stimmulierendem Coffein. Die meisten Sorten enthalten Monoterpene, die als wichtiger Rohstoff für viele naturheilkundliche Arzneimittel mit geringen Nebenwirkungen gelten. Verschiedene wissenschaftliche Arbeiten haben in den letzten Jahrzehnten die therapeutischen Effekte vieler ätherischer Öle der Sideritis-Arten belegt (z.B. stressmindernd).

Auf der Suche nach wirksamen Phyto-Wirkstoffen untersuchten Wissenschaftler aus dem Mittelmeerraum auch ihre einheimischen Pflanzen. An der Gazi-Universität in Ankara (Türkei) wurde beispielsweise ein langfristiges Programm zur Untersuchung der Sideritis-Gattung initiiert. Die Wissenschaftler überprüften die volksmedizinischen Ansätze und konnten tatsächlich nachweisen, dass verschiedene Sideritis-Arten antibakterielle, antioxidative, schmerzlindernde und entzündungshemmende Wirkung haben.

Es besteht jedoch kein oder nur ansatzweise eine dokumentierter Zusammenhang, welche definierte Species eine spezifische pharmakologische Aktivität aufweist. Insbesondere für die Beeinflussung des humanen Zentralnervensystems existieren keine Daten, welche Sideritis species in welcher Zubereitungsform besonders bevorzugt ist.

**Kognition** ist ein uneinheitlich verwendeter Begriff, mit dem auf die Informationsverarbeitung von Menschen und anderen Systemen Bezug genommen wird. Oft ist mit "Kognition" das Denken in einem umfassenden Sinne gemeint. Auch wenn viele kognitive Prozesse im Menschen bewusst sind, haben "Kognition" und "Bewusstsein" nicht die gleiche Bedeutung. So können bestimmte Prozesse im Menschen unbewusst und dennoch kognitiv sein, ein Beispiel hierfür ist das unbewusste Lernen. Zu den kognitiven Fähigkeiten eines Menschen zählen zum Beispiel die Aufmerksamkeit, die Erinnerung, das Lernen, die Kreativität, das Planen, die Orientierung, die Imagination, die Argumentation, die Selbstbeobachtung, der Wille, das Glauben und einige mehr. Kognitive Fähigkeiten werden von verschiedenen Wissenschaften, wie der Psychiatrie, der Psychologie, der Philosophie und den Neurowissenschaften untersucht.

Die kognitive Leistungsfähigkeit ist daher ein komplexer Prozess, der mit messbaren Parametern an Gehirnleistung quantifiziert werden kann.

Die Fähigkeit zu Lernen umfasst sowohl Memorieren (Dauer und Menge an behaltenem Input), als auch den Einfluss auf die Reaktionsgeschwindigkeit, die Fähigkeit logische Operationen (schnell und richtig) durchzuführen, oder räumliches Denkvermögen, z.B. in einer Orientierungsphase unter neuen oder veränderten Bedingungen. Störungen dieser kognitiven Leistungsfähigkeit werden unter anderem mit dem Syndrom MCI (Mild Cognitive Impairment) beschrieben. MCI stellt eine Sonderbedingung der altersbedingten Minderung kognitiver Funktionen und Fähigkeiten dar. Dieses Syndrom ist nicht nur durch den subjektiven Verlust an Merkfähigkeit charakterisiert; in der Regel sind auch andere kognitive Funktionssysteme (z.B. Aufmerksamkeit, exekutive Funktionen) betroffen. Die Überprüfung des MCI kann an gesunden Menschen als ein Präindikator für spätere Demenzen angesehen werden. Die letzte Phase kognitiver Leistungseinbußen führt dann zur absoluten Orientierungslosigkeit, sowohl räumlich als auch zeitlicher Natur (z.B. Alzheimer).

Die Elektroenzephalografie (von griechisch *encephalon* Gehirn, *gráphein* schreiben) ist eine Methode der medizinischen Diagnostik zur Messung der summierten elektrischen Aktivität des Gehirns durch Aufzeichnung der Spannungsschwankungen an der Kopfoberfläche. Das Elektroenzephalogramm (EEG abgekürzt) bietet als graphische Darstellung dieser Schwankungen eine standardmäßige Untersuchungsmethode in der Neurologie. Ursache dieser Potenzialschwankungen sind physiologische Vorgänge einzelner Gehirnzellen, die durch ihre elektrischen Zustandsänderungen zur Informationsverarbeitung des Gehirns beitragen. Entsprechend ihrer spezifischen räumlichen Anordnung addieren sich die von einzelnen Neuronen erzeugten Potenziale auf, so dass sich über den gesamten Kopf verteilte Potenzialänderungen messen lassen. Zur klinischen Bewertung wird eine Aufzeichnung in mindestens zwölf Kanälen von verschiedenen Elektrodenkombinationen benötigt. Die Ortsauflösung des üblichen EEGs liegt bei mehreren Zentimetern. Wenn eine höhere Ortsauflösung benötigt wird, so müssen die Elektroden nach neurochirurgischer Eröffnung des Schädels direkt auf die zu untersuchende Hirnrinde aufgelegt werden. Das ist jedoch nur in Sonderfällen z.B. vor epilepsiechirurgischen Eingriffen erforderlich. In diesem Falle spricht man von einem Elektrocorticogramm (ECoG), welches eine räumliche Auflösung von unter 1 cm ermöglicht und zusätzlich die Möglichkeit bietet, durch selektive elektrische Reizung einer der Elektroden die Funktion der darunterliegenden Hirnrinde zu testen. Eine noch detailliertere Erfassung von Einzelzellaktivität ist nur im Tierexperiment möglich. Die resultierenden Daten können von geübten Spezialisten auf auffällige Muster untersucht werden. Eine verbreitete mathematische Methode zur Analyse des EEGs ist die Fouriertransformation der Daten vom Zeitbereich (also der gewohnten Darstellung von Spannungsänderungen im Verlauf der Zeit) in dem sogenannten Frequenzbereich. Die so gewonnene Darstellung erlaubt die schnelle Bestimmung von rhythmischer Aktivität. Beim papierlosen oder Computer-EEG wird das Signal digitalisiert und vom Neurologen oder Psychiater meist am Bildschirm ausgewertet.

Die makroskopisch sichtbare elektrische Hirnaktivität kann Motive aufweisen, die rhythmischer Aktivität gleichen. Grundsätzlich gleicht das EEG jedoch dem frequenzabhängigen Rauschen (1/f-Rauschen) und enthält keine lang andauernden Oszillationen. Verschiedene Wachheitsgrade werden von Änderungen des Frequenz-Spektrums der EEG-Signale begleitet, so dass sich durch eine Analyse der gemessenen Spannungskurven vage Aussagen über den Bewusstseinszustand treffen lassen. Häufig wird das EEG in Frequenzbänder (sogenannte EEG-Bänder) eingeteilt, wobei die Anzahl von Bändern wie auch die genaue Einteilung z.T. unterschiedlich angegeben wird. Die Einteilung der Frequenzbänder und deren Grenzen sind historisch bedingt und decken sich nicht durchgehend mit Grenzen, die auf Grund modernerer Untersuchungen als sinnvoll gelten. So wurde beispielsweise das Theta-Band in einen Bereich Theta 1 und Theta 2 aufgeteilt, um den unterschiedlichen Bedeutungen der Teilbereiche Rechnung zu tragen. Die EEG-Auswertung erfolgt traditionell durch Mustererkennung des geschulten Auswerters. Insbesondere für Langzeit- und Schlaf-EEGs werden auch Software-Algorithmen zur assistierten oder automatischen Auswertung eingesetzt, die diese Mustererkennung nachbilden sollen. Dies gelingt einfacher für die hauptsächlich im Frequenzbereich definierten EEG-Bänder, etwas schwieriger für sonstige typische Muster im EEG. So deutet z.B. ein sehr asynchrones Muster aller Frequenzbänder auf starke emotionale Belastung oder Verlust der willentlichen Kontrolle hin, während vermehrt langsame Wellen bei gleichzeitig wenigen schnellen Wellen auf einen Schlaf- oder einen dösenden Zustand hinweisen.

Delta-Wellen weisen eine niedrige Frequenz von 1 bis 4 Hz auf. Sie sind typisch für die traumlose Tiefschlafphase. Delta-Wellen werden durch den Eingriff in das cholinerge beeinflusst.

Als Theta-Welle wird ein Signal im Frequenzbereich zwischen 4 und 7 Hz bezeichnet. Sie treten vermehrt in den leichten Schlafphasen auf und man reagiert nur noch auf wichtige oder starke Umweltreize. Theta Wellen werden durch Interaktionen mit dem noradrenergen alpha-2 Rezeptor verändert.

Als Alpha-Welle wird ein Signal im Frequenzbereich zwischen 8 und 13 Hz bezeichnet. Ein verstärkter Anteil von Alpha-Wellen wird mit leichter Entspannung, bzw. entspannter Wachheit, bei geschlossenen Augen, assoziiert. Alpha-Wellen treten hauptsächlich bei geschlossenen Augen auf und gehen mit dem Öffnen der Augen dann in den Beta-Bereich über. Den gleichen Effekt erreicht man bei geschlossenen Augen, wenn man z.B. eine einfache Rechenaufgabe im Kopf zu lösen beginnt. Man unterscheidet zwischen Alpha-1-und Alpha-2-Wellen. Alpha-1-Wellen scheinen unter serotonerger Kontrolle zu stehen, Alpha-2-Wellen verändern sich mit Änderungen der Aktivität des dopaminergen Systems.

Beta-Wellen nehmen einen Frequenzbereich zwischen 14 und 30 Hz ein. Das Auftreten von Betawellen hat verschiedene Ursachen und Bedeutungen, z.B. kommen Betawellen bei etwa 8% aller Menschen als normale EEG-Variante vor. Betawellen kommen u.a. auch im REM-Schlaf vor. Physiologisch treten β-Oszillationen außerdem z.B. beim konstanten Halten einer Kraft auf. Als Gamma-Welle wird ein Signal im Frequenzbereich über 30 Hz bezeichnet. Sie treten zum Beispiel bei starker Konzentration oder Lernprozessen auf. Neuere Forschungen zeigten die Bedeutung des Gammabandes bei der s.g. Top-Down Regulierung und der Synchronisation von verschiedenen Hirnarealen zur Integration verschiedener Qualitäten eines Stimulus. Man unterscheidet im EEG zwischen Änderungen vor allem der Beta-1- und Beta-2-Wellen. Änderungen der Beta-1-Wellen werden bei Eingriffen in das glutamaterge System beobachtet. Medikamente, die ins GABAerge System eingreifen produzieren Änderungen in den Beta-2-Wellen.

Gehirnwellen lassen sich aber nicht nur messen, sondern auch beeinflussen. Das kann durch die Reizung von Sinnesnerven geschehen (visuelle / akustische oder olfaktorische Reize) oder als Neurofeedback - eine Spezialform des Biofeedbacks - in Folge pharmakologisch aktiver Substanzen, wie Psychopharmaka [Dimpfel W, et al. (1996) Source Density Analysis of Functional Topographical EEG: Monitoring of Cognitive Drug Action. Eur J Med Res 1: 283 - 290]. Die Auswertung wird auch als Elektropharmakogram bezeichnet. Beim Neurofeedback ist es üblich, die EEG-Bänder feiner zu unterteilen und anders zu interpretieren als im klinischen EEG. Eine erhöhte Amplitude innerhalb der Frequenzbereiche wird mit gewissen mentalen Zuständen oder Aktivitäten korreliert. Theta-2-Wellen können z.B. mit Erinnerungs- und Lernfähigkeit, Konzentration, und 7 oder Kreativität in Zusammenhang gebracht werden. Ebenso können nach umfangreichen Kalibrierungen Rückschlüsse auf Neuro-transmitter-vermittelte ZNS-Aktivitäten gezogen werden, welche in dopaminerge, serotonerge, cholinerge oder noradrenerge Untergruppen eingeteilt werden können.

Ziel der vorliegenden Erfindung ist die Bereitstellung von weiteren Anwendungen von Zubereitungen und Extrakten der Gattung der Gliedkräuter (Sideritis ssp.), wie in den Ansprüchen definiert.

Die Aufgabe wird gelöst durch die erfindungsgemäße Verwendung von Extrakten der oberirdischen Pflanzenteile von Gliedkräutern (Sideritis ssp.) zur Steigerung kognitiver Leistungsfähigkeit, wobei es sich bei den oberirdischen Pflanzenteilen um frische oder getrocknete Teile von Pflanzen aus der Gruppe Sideritis euboa, Sideritis scardica, Sideritis raiseri oder deren Mischungen handelt, wie in den Ansprüchen definiert. Diese Extrakte können in Lebensmitteln, Nahrungsergänzungsmitteln, ergänzenden bilanzierten Diäten oder pharmazeutischen Zubereitungen eingesetzt werden.

Ötztürk, Y. und Aydin S. (Phytotherapy Research, Vol. 10, 70-73 (1996)) beschreiben für Zubereitungen verschiedener Sideritis-Species eine sedierende Aktivität, welche sie aus dem "Swimming perfomance test" ableiten. Dieser ist jedoch normalerweise ein Maß für die Motivationsfähigkeit bei depressiven Mäusen, oder ein Ausdauertest zur Effektmessung muskelstärkender Nährmittel. Einflüsse auf kognitive Fähigkeiten wurden nicht beschrieben.

In der Patentanmeldung EP 1 634 602 wird für Pflanzen der Gattung Sideritis eine Beeinflussung serotonerger Neurotransmission beschrieben. In dieser Schrift wurden jedoch leider keinerlei Angaben zur verwendeten Spezies offenbart, noch ein Hinweis gegeben, welchen Einfluss die Auswahl des Extraktionsmediums hat. Die in den Absätzen [0046] bis [0054] beschriebene Versuchsanordnung zur Messung serotonerger Reuptake-Raten wurde für eine Vorauswahl geeigneter Spezies für die eigenen Untersuchungen verwendet.

Die Erfindung beschreibt die Herstellung von Pflanzenextrakten aus Sideritis ssp. und deren Verwendung zur kognitiven Leistungssteigerung, wie in den Ansprüchen definiert.

Gemäß des Tiermodelles "Moris Water maze" wird die Steigerung der kognitiven Leistungsfähigkeit besonders unter bestehender Belastungssituation sichtbar, in dem eine Steigerung der Lernleistung insbesondere der Merkfähigkeit gezeigt wurde.

Die bestehende Belastungssituation wurde hierbei durch eine Stresssituation erzeugt, hätte jedoch auch durch eine neurodegenerative Erkrankung ausgelöst werden können. Im Humanversuch wurde die Belastungssituation durch Lernstress ausgelöst, insbesondere in Verbindung mit neuen Aufgaben in einer besonderen Situation, z.B. bei Prüfungsangst.

Entsprechend der durchgeführten Untersuchungen eignen sich oberirdische Pflanzenteilen von Pflanzen aus der Gruppe Sideritis euboa, Sideritis scardica, Sideritis raiseri unabhängig vom Trocknungsgrad der Droge. Eine Trocknung unterhalb von 12% erweist sich hinsichtlich der Lagerfähigkeit der Droge als besonders günstig, um Zersetzungsprozesse weitestgehend zu unterbinden und die mikrobiologische Belastung der Droge gering zu halten. Eine Zerkleinerung auf für die Extraktion günstige Größen um 1 cm verbessert die Wirkstoffausbeuten, insbesondere wenn die Stängelanteile nach einer Rebelung und anschließender Windsichtung abgereichert werden.

Das stärkste Aroma entfaltet Sideritis zum Zeitpunkt der Blüte. Für die Nutzung in einem Lebensmittel hat es daher als besonders geeignet erwiesen, wenn die Droge zum Zeitpunkt der Blüte geerntet wurde.

Sollte eine Mischung aus mehreren Subspecies verwendet werden, so hat sich ein möglichst großer Anteil an S.scardica als bevorzugt erwiesen, wenn insbesondere der Anteil an S.scardica mindestens die Hälfte beträgt, bevorzugt bei mindestens 80%.

Entsprechend der Erfahrungen aus dem Lösemittelscreenning eignen sich zur Extraktion besonders polare Auszugsmittel wie Wasser, ein- und mehrwertiger Alkohole oder Ketone, insbesondere Alkohole oder Ketone mit 1 bis 4 C-Atomen und besonders deren Mischungen mit Wasser. Ohne eine abschließende Aufzählung zu geben, dürfen als besonders geeignet folgende Auszugsmittel angesehen werden: Methanol, Ethanol, 1-Propanol, 2-Propanol, Propan-1,2-diol, Propan-1,3-diol, Glycerol, Dimethylketon, Methylethylketon. Auch die Verwendung von Zuckern (Mono-, Di- und Oligomere) sowie von niedermolekularen Polyethylenglykolen ist als Cosolvenz denkbar.

Das Extraktionsverfahren ist energetisch am besten bei Temperaturen maximal 10°C unterhalb der Siedetemperatur des Auszugsmittels durchzuführen, um den Systemdruck möglichst niedrig zu halten. Für die Aromen hat sich zudem eine Temperatur von maximal 100°C als am besten geeignet gezeigt. Um die Auszugsmittel mit möglichst wenig Druck zu fördern, hat sich eine Fördertemperatur von mindestens Raumtemperatur, also etwa 20°C, als am besten geeignet erwiesen.

Um den Umgang mit den Extrakten großtechnisch zu erleichtern, wird das Extraktionseluat möglichst auf einen Gehalt von über 50% Trockensubstanzanteil angereichert, in dem das Lösemittel entfernt wird. Besonders schonend und energieeffizient sind hierbei Verfahren mittels Unterdruck bei mäßigen Temperaturen von etwa 50°C.

Eine weiter verbesserte Handhabbarkeit und Lagerstabilität wird durch eine weitestgehende Trocknung erreicht. Diese wird gemäß dem Stand der Technik heutzutage bevorzugt mittels Gefriertrocknung, Sprühtrocknung, Bandtrocknung, Vakuumtrocknung, Walzentrocknung oder Kombinationen dieser Verfahren durchgeführt.

Die Verwendung der so hergestellten Extrakte ist in einem Lebensmittel, einem Nahrungsergänzungsmittel, einer ergänzenden bilanzierten Diät oder einer pharmazeutischen Zubereitung (Arzneimittel) denkbar.

Hierzu würde der Extrakt üblicherweise in eine dem Anwender gerechte Form überführt, wie z.B. einer Tablette, einer Kapsel, einer Kauzubereitung, einer Lutschzubereitung, einer Brausezubereitung, eines Granulates, eines Getränkes oder einer Instantzubereitung, insbesondere einer Instantteezubereitung.

Um die gewünschte Dosierung in einer solchen Form einzustellen werden üblicherweise physiologisch verträgliche Hilfsstoffe verwendet. Hierzu zählen unter anderem Kohlehydrate wie Stärkeabbauprodukte z.B. Maltodextrin, Glucosesirup, Zucker aber auch Zellulose und die entsprechenden Kohlehydrat-Derivate. Des Weiteren verwendet man aus der Gruppe der Proteine beispielsweise Gummi arabicum, Gelatinen oder Kollagenhydrolysate.

Wird eine Verwendung in einer therapeutischen Blickrichtung angestrebt, so wäre auch eine synergistisch wirkende Kombination mit Nootropika möglich, die z.B. auch in der Demenz-Therapie Anwendung finden. Mögliche Kombinationspartner wären Acetylcholinesterase-Hemmer (z.B. Donezepil, Galantamin, Rivastigmin, Tacrin und deren Derivate), GABA-Analoga (z.B. Piracetam und Derivate), Ergot-Derivate (z.B. Nicergolin, Dihydroergotoxin und Derivate) und NMDA-Antagonisten (Memantin).

Zur Herstellung eines Extraktes aus Sideritis ssp. hat sich ein Verfahren als besonders geeignet erwiesen, welches die folgenden Schritte umfasst:
a) ausgewählt werden aus den Species Sideritis euboa, Sideritis scardica, Sideritis raiseri oder deren Mischungen, die oberirdischen Pflanzenteile,
b) welche zum Zeitpunkt der Blüte geerntet werden,
c) optional auf eine Restfeuchte von <12% getrocknet werden,
d) optional durch Schneiden auf ca. 1 cm und Windsichtung die Stängel weitestgehend abgetrennt werden,
e) mit einem Auszugsmittel, ausgewählt aus der Gruppe Wasser, ein- und mehrwertiger Alkohole und Ketone, insbesondere Alkohole oder Ketone mit 1 bis 4 C-Atomen oder deren Mischungen, extrahiert werden bei
f) einer Auszugsmitteltemperatur zwischen 20 und 100°C, bevorzugt bei Temperaturen 10°C unterhalb der Siedetemperatur des Auszugsmittels,
g) das Auszugsmittel zumindest teilweise entfernt wird, bevorzugt schonend mittels Unterdruck,
h) das ätherische Öl im Unterdruck auf kleiner 0,1% im Extrakt abgereichert wird,
i) der daraus resultierende Extrakt getrocknet wird, bevorzugt mit Gefriertrocknung, Sprühtrocknung, Bandtrocknung, Vakuumtrocknung, Walzentrocknung oder einer Kombinationen dieser Verfahren.

Figur 1 zeigt die Wirkung des Sideritis-Trockenextraktes nach Beispiel 5 MEEG Tiermodel; vgl. Beispiel 7.
Figur 2 zeigt die Wirkung des Sideritis-Trockenextraktes nach Beispiel 5a.
Figur 3 zeigt ein "Brain Map" nach Gabe von Sideritis-Trockenextrakt; vgl. Beispiel 9.
Figur 4 zeigt die Ergebnisse im Water-Maze-Test gemäß Beispiel 11.
Figur 5 fasst die Ergebnisse aus Beispiel 11 zusammen.
Figur 6 zeigt die Wirkung von Sideritis-Extrakt gemäß Beispiel 12.

Die Erfindung wird durch die folgenden Beispiele näher erläutert:

### Beispiel 1: Herstellung teeanaloger Extrakte aus Herba Sideritis

50 g Ausgangsdroge werden zweimal mit der 15fachen Menge kochenden Wassers versetzt und unter Rühren extrahiert. Beide Mazerationsauszüge werden zum Abkühlen auf Raumtemperatur stehengelassen und vereinigt. Die beiden vereinigten Mazerate werden über einen Faltenfilter abfiltriert und am Rotationsverdampfer zum Spissum eingeengt. Hierbei ergaben sich für die folgenden Spezies die ermittelten Extraktivstoffausbeuten. Zur Testung auf die Serotoninaufnahme-Inhibierung wurden die Extrakte alle auf den gleichen Nativanteil eingewogen und mit einer Meßkonzentration von 50 µg/ml im Testsystem gemäß EP 1 634 602 untersucht.

Eine Negativ-Kontrolle mit Serotonin ergibt 0%; eine Positiv-Kontrolle mit (10 µM) der Referenzsubstanz Fluvoxamin 100%.

| Spezies | Extraktivstoff-Ausbeute [%] | Serotoninaufnahme-Inhibierung [rel. % vs. Kontrolle] |
|---|---|---|
| *Sideritis congesta** | 22,8 | 5 |
| *Sideritis vuralii** | 25,9 | 10 |
| *Sideritis argyrea** | 25,3 | 12 |
| *Sideritis arguta** | 17,0 | 17 |
| *Sideritis pisidica** | 19,3 | 12 |
| *Sideritis scardica* | 15,7 | 39 |
| *Sideritis raiseri* | 17,1 | 31 |
| *Sideritis euboa* | 1.7,2 | 28 |

| | | |
|---|---|---|
| *(Vergleichsbeispiel) | | |

Entsprechend dem Habitus der einzelnen Species (verschiedene Stängel und Blattanteile) variieren die Ausbeuten zwischen rd. 16-26 %. Dabei gehen hohe Extraktausbeuten wie bei Sideritis vuralii mit einer niedrigen Aktivität einher. Die Species S. scardica, S. raiseri und S. euboa mit einer vergleichsweise niedrigen Ausbeute sind bezüglich der Serotoninaufnahme-Inhibierung favorisiert.

### Beispiel 2: Herstellung erfindungsgemäßer hydroalkoholischer Extrakte aus Herba Sideritis

50 g Herba *Sideritis* wurden zweimal mit der 15fachen Menge einer Mischung aus Wasser und Ethanol bei 45°C versetzt und unter Rühren extrahiert. Beide Mazerationsauszüge werden zum Abkühlen auf Raumtemperatur stehengelassen und vereinigt. Die beiden vereinigten Mazerate werden über einen Faltenfilter abfiltriert und am Rotationsverdampfer zum Spissum eingeengt. Hierbei ergaben sich für die folgenden Spezies die ermittelten Extraktivstoffausbeuten. Zur Testung auf die Serotoninaufnahme-Inhibierung wurden die Extrakte alle auf den gleichen Nativanteil eingewogen und mit einer Meßkonzentration von 50 µg/ml im Testsystem gemäß EP 1 634 602 untersucht.

Eine Negativ-Kontrolle mit Serotonin ergibt 0%; eine Positiv-Kontrolle mit (10 uM) der Referenzsubstanz Fluvoxamin 100%.

| Spezies | Auszugsmittel + Konzentration | Serotonin-Aufnahmeinhibierung [rel. % vs. Kontrolle] |
|---|---|---|
| Siederitis *euboa* | 20% V/V Ethanol | 58 |
| Siederitis scardica | 20% V/V Ethanol | 65 |
| Sideritis pisidica* | 20% V/V Ethanol | 28 |
| Sideritis pisidica* | 30% V/V Ethanol | 64 |
| Sideritis pisidica* | 50% V/V Ethanol | 25 |
| Sideritis pisidica* | 70% V/V Ethanol | 19 |

| | | |
|---|---|---|
| *(nicht erfindungsgemäß) | | |

Am Beispiel der Species S. pisidica wurde der Einfluss des Auszugsmittels untersucht. Eine Erhöhung des Ethanolanteils auf 20% V/v gegenüber reinem Wasser führt zu einer 2,3fach stärkeren Aktivität. Bereits eine um nur 10% V/V erhöhte Auszugsmittelstärke von Ethanol 30% V/V ergab eine um 5,3 erhöhte Aktivität. Weitere Ethanolerhöhungen ergaben keine weiteren Steigerungen, vielmehr ist sogar ein deutlicher Abfall für Ethanol 50% V/V bzw. Ethanol 70% V/V zu messen. Eine Ethanolkonzentration von 20 - 30% V/V Ethanol ist eine bevorzugte Extraktionsstärke. Dies wird auch an den gemäß Beispiel 1 favorisierten Species sichtbar, wo für S. scardica eine um den Faktor 1,7 und für Sideritis euboa eine um den Faktor 2,0 erhöhte Aktivität nachweisbar ist.

### Beispiel 3: Herstellung eines erfindungsgemäßen hydroalkoholischen Extraktes aus einer Frischpflanze

100 g frischer Droge aus Herba *Sideritis scardica* wurde zweimal mit der 15fachen Menge (V/V) 20%-igen Ethanols bei 45°C versetzt und perkoliert. Beide Perkolate wurden zum Abkühlen auf Raumtemperatur stehengelassen, über Faltenfilter abfiltriert und am Rotationsverdampfer zum Spissum eingeengt.

Hierbei ergab sich die folgend genannte Serotonin-Aufnahmeinhibierung bei einer Meßkonzentration von 50 µg/ml im Testsystem gemäß EP 1 634 602:

| Spezies | Auszugsmittel + Konzentration | Serotonin-Aufnahmeinhibierung [rel. % vs. Kontrolle] |
|---|---|---|
| Sideritis *scardica* Frischpflanze | 20% V/V Ethanol | 45 |

### Beispiel 4: Herstellung eines erfindungsgemäßen hydroalkoholischen Extraktes aus einer getrockneten, vorbehandelten Sideritis-Rohware

Herba *Sideritis scardica* wurde frisch geerntet und per Wärmeumluftrocknung binnen 7 Tagen am Stück getrocknet. Der Restfeuchteanteil lag bei 10,2%.

Die so erhaltene Ware wird gebündelt und als Teeware eingesetzt.

Um die Verwendung zur Extraktion zu gewährleisten, wird üblicherweise eine geschnittene 2-5 cm lange Herba-Ware eingesetzt. Eine Optimierung des

Extraktivstoffgehaltes wird von geschnittener Ware (1 cm) erwartet. Eine besondere Ausführungsform liegt in der Rebelung der Herba Sideritis Ware. Dadurch werden maschinell Blätter und Blüten-Anteile vom Stängel getrennt. Mit anschließender Windsichtung kann der Stängelanteil auf <5% reduziert werden.

Die drei verschieden aufbereiteten Rohwaren von Sideritis scardica wurden nach der gemäß Beispiel 2 beschriebenen Weise mit (V/V) 20%-igen Ethanol extrahiert. Hierbei ergab sich die folgende Serotoninaufnahme-Inhibierung bei einer Meßkonzentration von 50 pg/ml im Testsystem gemäß EP 1 634 602:

| Sideritis *scardica getrocknete Rohware* | Extraktivstoff-Ausbeute [%] | Serotoninaufnahme-Inhibierung [rel. % vs. Kontrolle] |
|---|---|---|
| 6-8 cm | 16,2 | 65 |
| 1 cm normaler Stängelanteil | 17,5 | 64 |
| 1cm reduzierter Stängelanteil | 21,3 | 64 |

Die Versuche belegen, dass die Vorbehandlung der Rohware eine Ausbeute-Erhöhung um relative 31% ergab, wobei die pharmakologische Aktivität unverändert blieb.

### Beispiel 5: Herstellung einer Sideritis-Extraktzubereitung

a) 10 kg Herba *Sideritis scardica L.* wurde in einem Extraktionsansatz mit 300 Litern (V/V) 20%-igem Ethanol bei 45°C in einem Perkolator während 8 Stunden extrahiert und über einen 250 µm-Siebbeutel von der Droge befreit. Das Eluat wurde auf Raumtemperatur abkühlen gelassen und über einen Zellulosefilter geklärt. Anschließend wurde im Unterdruck zu einem Spissum (Dickextrakt) mit einem Trockensubstanzgehalt von ca. 50% eingeengt. Die Extraktausbeute betrug 16%, was einem nativen Droge-Extrakt-Verhältnis von 6:1 entspricht. Für den Trocknungsansatz wurde 70% nativer Extrakt mit 30% Maltodextrin als Träger versehen und im Vakuum bei 50°C getrocknet.
b) 10 kg Herba *Sideritis euboa L.* wurde in einem Extraktionsansatz mit 300 Litern (V/V) 20%-igem Ethanol bei 45°C in einem Perkolator während 8 Stunden extrahiert und über einen 250 µm-Siebbeutel von der Droge befreit. Das Eluat wurde auf Raumtemperatur abkühlen gelassen und über einen Zellulosefilter geklärt. Anschließend wurde im Unterdruck zu einem Spissum (Dickextrakt) mit einem Trockensubstanzgehalt von ca. 50% eingeengt. Die Extraktausbeute betrug 17%, was einem nativen Droge-Extrakt-Verhältnis von 6:1 entspricht. Für den Trocknungsansatz wurde 70% nativer Extrakt mit 30% Maltodextrin als Träger versehen und im Vakuum bei 50°C getrocknet.

Die beiden resultierenden Trockenextrakte a) und b) wurden jeweils zu einem homogenen Extrakt-Pulver über einem 1 mm Sieb gemahlen. Die Mischung eines Sideritis scardica-Trockenextraktes (a) mit einem Sideritis euboa-Trockenextrakt (b) im Verhältnis 1:1 ergibt eine erfindungsgemäße Zubereitung.

### Beispiel 6: Herstellung einer Sideritis-Extraktzubereitung

10 kg Herba *Sideritis scardica L.* wurde in einem Extraktionsansatz mit 300 Litern (V/V) Wasser bei 80°C in einem Perkolator während 8 Stunden extrahiert und über einen 250 µm-Siebbeutel von der Droge befreit. Das Eluat wurde auf Raumtemperatur abkühlen gelassen und über einen Zellulosefilter geklärt. Anschließend wurde im Unterdruck zu einem Spissum (Dickextrakt) mit einem Trockensubstanzgehalt von ca. 50% eingeengt. Die Extraktausbeute betrug 17,5%, was einem nativen Droge-Extrakt-Verhältnis von 6:1 entspricht. Für den Trocknungsansatz wurde 70% nativer Extrakt mit 30% Maltodextrin als Träger versehen und im Vakuum bei 50°C getrocknet.

### Beispiel 7: Tele-Stereo-EEG-Messung an freibeweglichen Ratten - 1 mit der Zubereitung nach Beispiel 5

Einer Gruppe von n=8 Fischer-344-Ratten wurden je 4 Semimicroelectroden in die 4 Hirnareale "frontaler Cortex", "Hippocampus", "Striatum" und "Formatio reticularis" implantiert. Die messbaren Potentialfeldwechsel wurden durch Funk übertragen und wurden zu einem Elektropharmakogramm ausgewertet. Die Tiere wurden mit Sideritis-Trockenextraktmischung (aus Beispiel 5) in drei unterschiedlichen Dosierungsäquivalenten (50, 100, 200 mg/kg KG oral) in einem Cross-over-Design exponiert. Jede Einzeldosierung wurde hierzu in Wasser gelöst und einmalig nach je einer Woche "wash-out" verabreicht. Als Kontrollexperiment diente eine isotonische Kochsalzlösung. Nach einer 45-minütigen Prä-Drug-Beobachtungsphase wurde die Testflüssigkeit geschlündelt, gefolgt von einer 5-minütigen Beruhigungsphase für das Tier. Anschließend wurde die Messung über einen Meßzeitraum von 5 Stunden gestartet. Die Frequenzdaten wurden mittels Fast Fourier Transformation (FFT) erhalten und in 60-minütigen Perioden gemittelt. Die statistische Auswertung erfolgte mittels Wilcoxon, Mann and Whitney U-test gegen die Kontrolle (Salzlösung).

Alle Dosierungen ergaben vergleichbare Frequenzmuster. Die größten Effekte konnten bei den alpha-2-Wellen gesehen werden, welche für eine dopaminerge Nerotransmission stehen. Auch die dem glutaminergen System zugeordneten beta-1-Wellen konnten bereits bei der Dosierung von 50mg/kg KG signifikant von der Kontrolle unterschieden werden. Die Delta-, Theta- und speziell ab höheren Meßkonzentrationen auch die alpha-1-Wellen wurden ebenfalls abgedämpft, was im Hinblick auf die Aktivierung cholinerger, noradrenerger und serotonerger Neurotransmission zusammenpasst.

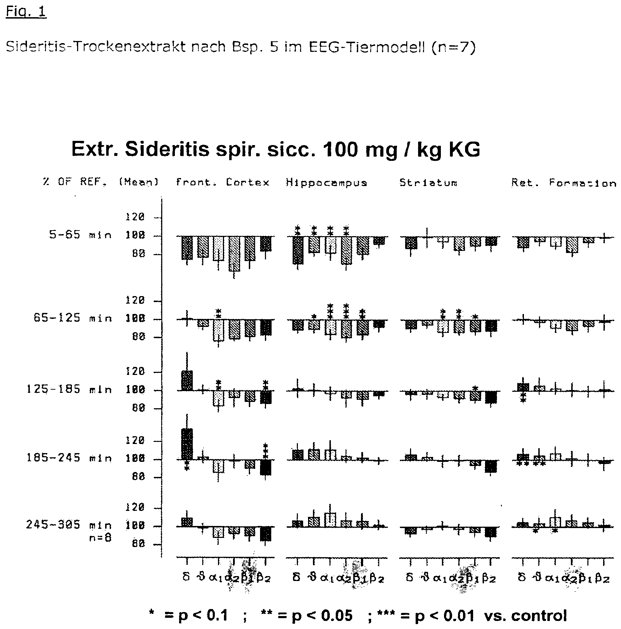

Ergebnisse siehe Figur 1.

### Beispiel 8: Tele-Stereo-EEG-Messung an freibeweglichen Ratten - 2 mit Sideritis scardica -Trockenextrakt aus Beispiel 5a

Analog des Versuchsaufbaus aus Beispiel 7 wurde das Meßmodell verwendet zur Messung der Beeinflussung des Elektropharmakograms mit dem Sideritis scardica-Trockenextrakt aus Beispiel 5a vor der Mischung. Die Dosierung wurde parallel ausgewählt (50, 100, 200 mg/kg KG oral). Jede Einzeldosierung wurde wieder in Wasser gelöst und einmalig nach je einer Woche "wash-out" verabreicht. Als Kontrollexperiment diente ebenfalls eine isotonische Kochsalzlösung.

Alle Dosierungen ergaben vergleichbare Frequenzmuster im frontalen Cortex, welcher für die Lernfähigkeit besonders beachtet wird. Die größten Effekte konnten erneut bei den alpha-2-Wellen gesehen werden, welche für eine dopaminerge Nerotransmission stehen. Auch die dem glutaminergen System zugeordneten beta-1-Wellen waren deutlich von der Kontrolle zu unterscheiden.

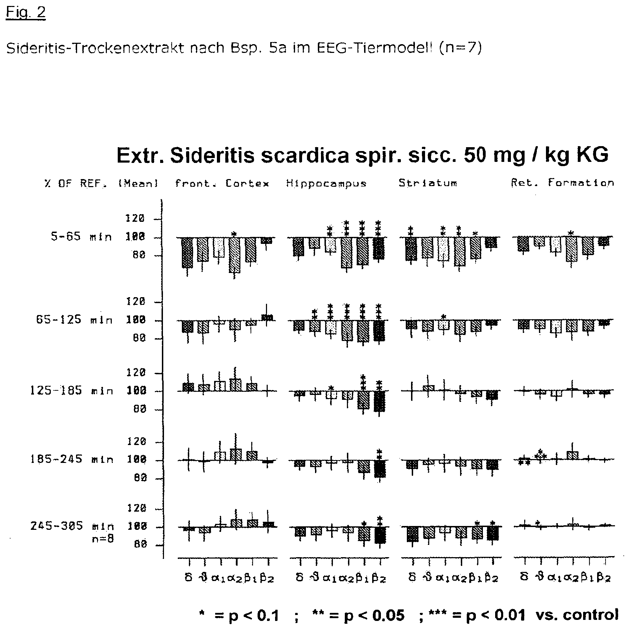

Ergebnisse siehe Figur 2.

### Beispiel 9: EEG-Messung in doppelblinder randomiserter placebokontrollierter Humanstudie im Cross-Over-Design zur Bewertung einer Einzelgabe

Von der Prüfmischung (Beispiel 5) wurden Kapseln á 400mg abgefüllt, von denen jeweils 3 Kapseln in der Studie als Einzeldosis zusammengehören.

Die Studie wurde durchgeführt, um eine evtl. vorhandene Leistungssteigerung im kognitiven Bereich nach einer akuten Einnahme eines Sideritis-Extraktes nachzuweisen. Hierzu wurden mit Hilfe eines interaktiven Fragetestes Probanden mit einer milden kognitiven Störung rekrutiert (DemTect Score 9-13) [Kessler J, et al. (2000) DemTect. Ein neues Screening-Verfahren zur Unterstützung der Demenzdiagnostik. Psycho 26: 343-7]. Eine Gruppe von n=18 ansonsten gesunden Probanden im Alter zwischen 40 und 65 Jahre wurde entsprechend klassifiziert und im Anschluss auf die Auswirkungen des Extraktes gemäß Beispiel 5 untersucht.

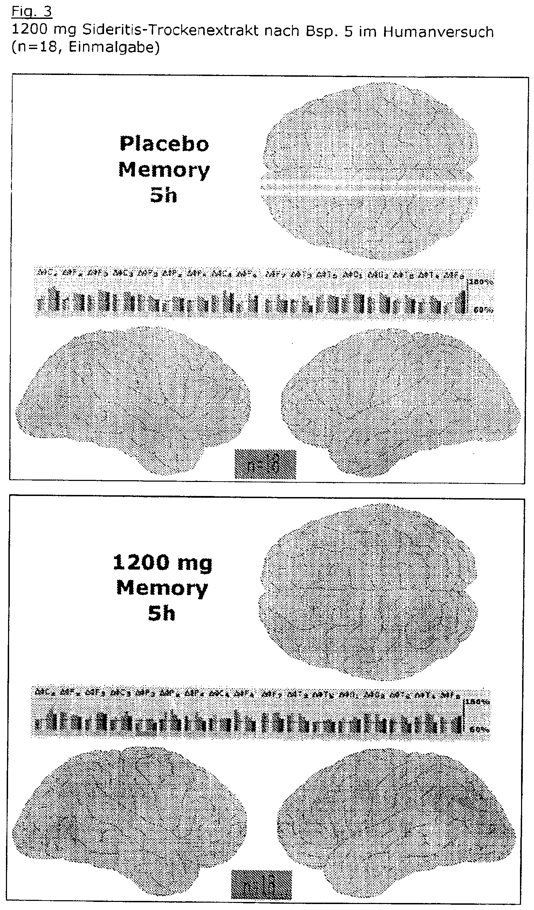

Das Elektroenzephalogramm der Probanden wurde unter relaxierten Bedingungen sowie während der Durchführung von drei verschiedenen kognitiven Tests abgeleitet (Elektropsychogramm). Sowohl im relaxierten Zustand als auch bei der Durchführung des Gedächtnistests konnten Veränderungen der elektrischen Leistung im Vergleich zur Placebo-Einnahme gemessen werden. Im relaxierten Zustand kam es zu einem Abfall der alpha Wellen (signifikant für alpha-2-Wellen in der letzten Stunde mit p<0.07). Veränderungen im Vergleich zu Placebo sind in Figur 3 als "Brain Map" dokumentiert. Während der Durchführung des Gedächtnistests wurde eine Zunahme der langsamen delta und theta Wellen, dagegen ein Abnahme der schnelleren alpha und beta Wellen beobachtet. Statistisch signifikant waren die Zunahme der alpha-1-Wellen (5. Stunde nach Einnahme; p<0.02) sowie die Abnahme der beta-2-Wellen (1. Stunde nach Einnahme; p<0.07). Auch in der Gehirnkarte wurde eine Zunahme der frontalen langsamen Wellen sichtbar. Eine Zunahme der langsamen Wellen bei gleichzeitiger Abnahme der schnellen Wellen ist charakteristisch für mentale Arbeit.

Die Ergebnisse der neurophysiologischen Datenanalyse geben einen ersten Hinweis für eine stimulierende Wirkung des Extraktes (Unterdrückung der zentralen alpha-2 Aktivität) mit Veränderungen der elektrischen Aktivität während der Durchführung des Gedächtnistests, die im Sinne einer besseren Leistung interpretiert werden können (Zunahme der frontalen delta und theta Wellen, stärkerer Abfall der zentralen alpha-2- und beta-1-Wellen). Die Einmaleinnahme lässt daher eine Steigerung der mentalen Gedächtnisleistung erkennen. Der Extrakt wurde sehr gut vertragen, Nebenwirkungen traten nicht auf.

### Beispiel 10: Fallbeispiel - Tee trinken und EEG messen

In einem Selbstversuch eines 42-jährigen Mannes konnte der Verzehr von rund 500 ml eines gekühlten Sideritis scardica-Tees mittels EEG überwacht werden. Hierbei waren sowohl alpha-2-Wellen gedämpft, als auch Delta- und Theta-Wellen angeregt. Die nach der Einnahme durchgeführten Merkfähigkeitstests, als auch die Konzentrationsfähigkeit waren signifikant verbessert.

### Beispiel 11: Verhaltensuntersuchungen im Moris Water Maze (MWM)

In einem mit trübem Wasser gefüllten runden Becken, welches seitlich mit ausgeprägten Markierungen versehen ist, sog. externen Hinweisreizen, werden Versuchstiere, in diesem Fall Mäuse, über mehrere Tage hinweg trainiert, selbstständig eine unter der Wasseroberfläche befindliche, nicht sichtbare Plattform zu finden und sich deren räumliche Position zu merken. Hierbei werden die Mäuse in etwa einem Abstand von ca. 30 cm vom Rand ins Wasser gelassen, woraufhin die Tiere umgehend mit Schwimmbewegungen versuchen die rettende Plattform zu erreichen. Der Vorteil dieses seit Beginn der 80er Jahre bekannten Messsystems gegenüber herkömmlichen einfachen Labyrinthen im Tierexperiment besteht darin, dass es keine lokalen Landmarken gibt, sondern nur globale und dass die Aufgabe aufgrund des Fluchtverhaltens der Tiere einen hohen Motivationsfaktor aufweist. Das Experiment zielt vor allem auf die Untersuchung des (räumlichen) Lernens (Erkennen und Memorieren) der Tiere unter Stressbedingungen und der Messung möglicher Einflüsse auf dieses. Messparameter sind hierbei die Zeit bis zum Auffinden der Plattform, die bis dorthin zurückgelegte Wegstrecke, sowie die relative Aufenthaltszeit im richtigen Quadranten des Beckens. Diese Parameter werden durch den Trainingseffekt beeinflusst. So reduziert sich üblicherweise Auffindzeit und Wegstrecke und die Quadrantenaufenthaltszeit verlängert sich. Durch unterschiedliche Neurotransmitterkonzentrationen kann der Trainingseffekt darüber hinaus beeinflusst werden [Dissertation, Uni Freiburg 2004, Theresa Schweizer: 3,4-Diaminopyridin evozierte Freisetzung von Neurotransmittern aus Hirnschnitten von Ratten: Untersuchungen im Kortex und Hippocampus an alten Ratten, sowie an Ratten mit serotonergen Läsionen hippocampaler Afferenzen und intrahippocampalen Raphé-Transplantaten].

In dieser Versuchsanordnung wurden 4 Gruppen á 6 Mäuse untersucht. Die erste Kontrollgruppe wurde von mit Wasser behandelten transgenen Tieren (Stamm APPS1 +/0) gebildet, welche auf Grund ihrer genetischen Disposition innerhalb von 50 Tagen nach ihrer Geburt eine starke β-Amyloidablagerung manifestieren, respektive an Alzheimer erkranken. Die zweite Kontrollgruppe bilden gesunde Vergleichsmäusen (Kontroll-Stamm APPPS1 0/0) ohne die entsprechende Genmutation. Die dritte Gruppe bilden transgene Tiere (Stamm APPS1 +/0), welche ab ihrem 50.sten Lebenstag mit einer Sideritis-Extraktlösung (aus Beispiel 5 geschlündelt wurden. Die vierte Gruppe wird von transgenen Tiere (Stamm APPS1 +/0) gebildet, welche ab ihrem 50.sten Lebenstag mit einer Extraktlösung von Ginkgo biloba (hergestellt entsprechend der europäischen Pharmakopoe) gleicher Konzentration behandelt (geschlündelt) wurden. Diese Gruppe wurde ausgewählt, da es sich bei Ginkgo biloba-Extrakten um die am stärksten verwendete Medikation für diese Thematik handelt.

Im Alter von ca. 95 Tagen startet eine verhaltensbiologische Testung mittels Morris Water Maze (95-100d). Der Test beinhaltet eine tägliche frühe und späte Test/Lerneinheit über vier Tage. Die frühe Einheit beginnt mit einem Lauf ohne Plattform für 30 Sekunden, hierbei wird die Zeit aufgezeichnet, in der sich die Maus in dem Quadranten aufhält, in dem sich gewohnter Weise die Plattform befindet (Zielquadrant). Die weiteren vier Läufe erfolgen mit unsichtbarer Plattform und mit 4 variierenden Startpositionen.

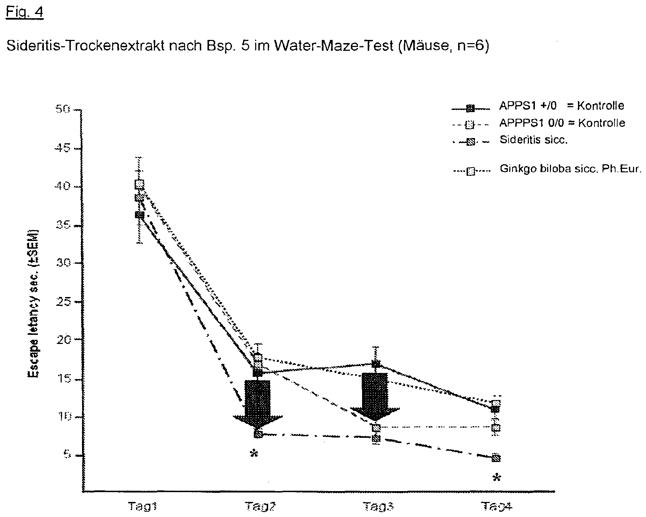

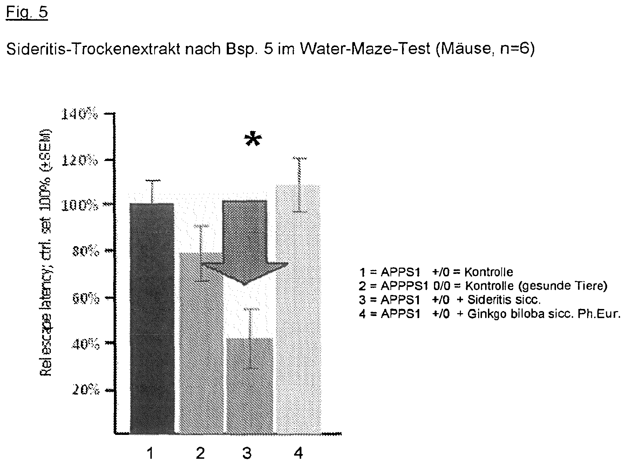

Analysiert wurden zwei Parameter: zum einen die Zeit bis zum Erreichen der Plattform (escape letancy, Figuren 4 und 5) zum anderen die Zeit, die die Mäuse im ersten Lauf des letzten Tages im Zielquadranten verbracht haben. Beide Parameter zeigen, dass der Ginkgo-Extrakt (Figur 4 = gepunktet, Figur 5 = Nr. 4) verglichen mit der transgenen Kontrolle (Figur 4 = durchgezogen, Figur 5 = Nr. 1) keinen Einfluss auf die kognitive Leistung in diesem Mausmodell hat. Hingegen kommt es bei einer Behandlung mit Sideritis-Extraktmischung zu einer beeindruckenden Steigerung der Gedächtnisleistung. So sind die mit Sideritisextrakt (aus Beispiel 5) behandelten Tiere (Figur 4 = gestrichelt, Figur 5 = Nr. 3) sowohl bereits an Tag 2, als auch an Tag 3 und 4 signifikant schneller im Erreichen der Plattform. Selbst im Vergleich zu den "gesunden Tieren" führte die Behandlung zu einer signifikanten Steigerung der Lernleistung an Tag 2 und 4.

### Beispiel 12: Verhaltensuntersuchungen (gesunder Mäuse) im MWM

Im Versuchsmodell Morris Water Maze analog Beispiel 11 startet im Alter von ca. 95 Tagen eine verhaltensbiologische Testung (95-100d). Es werden n=6 Tiere gemäß der zweiten Kontrollgruppe (Kontroll-Stamm APPPS1 0/0), mit einer parallelen Versuchsgruppe n=6 (Stamm APPPS1 0/0), welche ab ihrem 50.sten Lebenstag mit einem Sideritis-Extrakt (aus Beispiel 5) behandelt wurden, verglichen. Die Escape-Latency, als auch die Zeit im Zielquadranten zeigte eine beeindruckende Steigerung der Gedächtnisleistung an Tag 2, 3 und 4.

Nach weiteren etwa 35 Tagen wurde der Versuchsaufbau erneut für einen Vergleich beider Test-Populationen genutzt (d135 - d150). Die Verumpopulation wurde wieder für 15 Tage mit (12g/kgKG) Sideritisextrakt (Beispiel 5) behandelt. Es resultierte eine um 53% verminderte "Escape latency" an Tag 4 der Verum-Population, als auch einer Erhöhung der Zeit im Zielquadranten um über 30%.

Dieser Versuchsaufbau wurde nach weiteren 150 Tagen wiederholt (d275-d300), bei der die Verum-Population zuvor für 25 Tage mit 6g/kgKG Sideritisextrakt (Beispiel 5) behandelt wurde. Selbst nach dieser langen Zeit konnte noch eine signifikante Verminderung der escape latency an Tag 1 um 40% beobachtet werden.

An beiden Kollektiven wurde im Anschluss überprüft, ob auch ein erneuter Lernprozess von der kurzzeitigen Einnahme des Sidertitis-Extraktes profitieren könnte. Hierzu wurde die Plattform in einen anderen Quadranten verlegt. An Tag 1 gab es daher keinen messbaren Unterschied in der escape latency, jedoch zeigte sich ein Vorsprung der Verum-Population bereits ab Tag 2, und konnte bis Tag 4 auf 47% gesteigert werden.

Dieser Prozess des erneuten Lernens wurde abermals im Alter von d435 - d450 (entspricht einem Menschenalter von etwa 90 Jahren) untersucht. Wieder wurde die Verum-Population zuvor für 15 Tage mit 6g/kgKG Sideritisextrakt (Beispiel 5) behandelt. Auch hier konnte eine Reduktion der escape latency zunächst im Ursprungszielquadranten um 34%, als auch nach der Umstellung im neuen Zielquadranten an Tag 4 um 41% beobachtet werden.

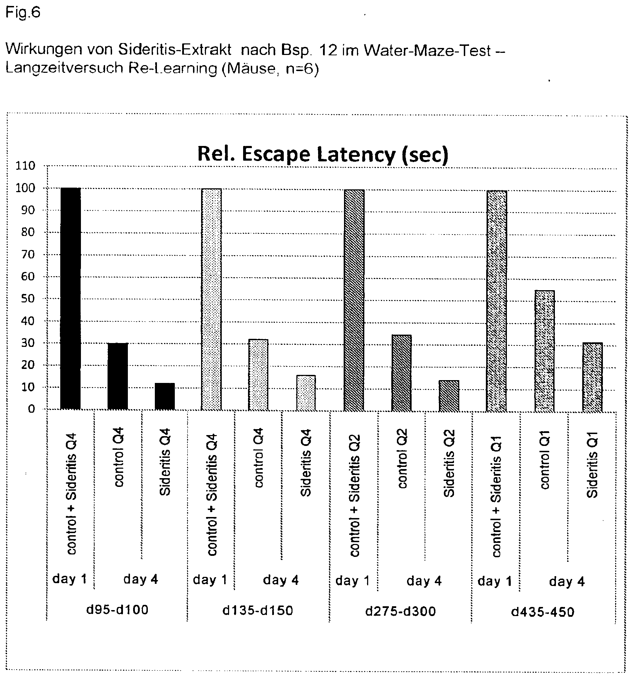

Die Ergebnisse sind in Figur 6 gezeigt.

### Beispiel 13: Schultee

Auf Grund des eigenen aromatischen Geschmackes kann Sideritis scardica als wässriger Teeauszug in leicht gesüßter Form als "Schultee" in einem klassischen Tetrapack in den Handel gebracht werden. Das Trockenextraktäquivalent sollte dabei zwischen 0,2g und 2g pro 100ml Teegetränk liegen. Als Süßungsmittel eignen sich sowohl klassische Zucker wie Fructose, Glucose oder Saccharose, aber auch Zuckeraustauschstoffe, wie Natriumsaccharat, Aspartam, Sucralose, Steviosid oder vergleichbare. Bereits kurze Zeit nach der Einnahme sind Steigerungen der kognitiven Leistungen zu erwarten, da analog Beispiel 9 und 10 die Wellenmuster im EEG beeinflusst werden.

Eine beispielhafte Zusammensetzung für einen Schultee lautet:
2,5 g Sideritis scardica Extrakt nach Beispiel 6
0,8 g Fructose
Wasser ad 200 ml

### Beispiel 14: Instanttee

2 kg eines Spissum-Extraktes aus Sideritis scardica, hergestellt entsprechend des in Beispiel 6 beschriebenen Verfahrens, wird mit Geschmackskorrigenzien (250 g Glucose, 10g Vitamin C, 1 g Sideritis-Flüssigaroma, 0,8 g Sucralose) versetzt und homogenisiert. Anschließend wird der Ansatz bei 180°C Zulufttemperatur sprühgetrocknet. Ein Meßlöffel dieses Pulvers kann mit 150ml kaltem Wasser zurückgelöst werden und ist somit direkt gebrauchsfertig.

### Beispiel 15: Brauseformulierung im Sacchet

Zur Herstellung der Brausetabletten werden 550 g Zitronensäure mit 300 g Natriumhydrogencarbonat, 50 g Fructose und 100 g des erfindungsgemäßen Extraktes aus Sideritis scardica gemäß Beispiel 6 gemischt. Nach erfolgter Homogenisierung kann das Gemisch granuliert oder direkt zu Tabletten verpresst werden. Als Einzeldosierung werden 4 g Granulat oder eine 4 g Brausetablette nach Bedarf 2x täglich empfohlen (= 2 x 400 mg Extrakt).

## Patentansprüche

1. Extrakt aus oberirdischen Pflanzenteilen von Sideritis ssp. zur Verwendung zur Steigerung kognitiver Leistungsfähigkeit, wobei es sich bei den oberirdischen Pflanzenteilen um frische oder getrocknete Teile von Pflanzen aus der Gruppe Sideritis euboa, Sideritis scardica, Sideritis raiseri oder deren Mischungen handelt, wobei der Extrakt getrocknet ist, ausgenommen zur Verwendung bei mild cognitive impairment bei Morbus Alzheimer und ausgenommen zur nicht-medizinischen Verwendung.

2. Extrakt zur Verwendung nach Anspruch 1, **dadurch gekennzeichnet, dass** es sich bei der Steigerung der kognitiven Leistungsfähigkeit um eine Steigerung der Lernleistung handelt, bevorzugt um eine Steigerung der Merkfähigkeit, besonders bevorzugt unter bestehender Belastungssituation.

3. Extrakt zur Verwendung nach Anspruch 2, **dadurch gekennzeichnet, dass** es sich bei der bestehenden Belastungssituation um Stresssituationen oder neurodegenerative Erkrankungen handelt, insbesondere um Lernstress in Verbindung mit neuen Aufgaben oder in besonderen Situationen.

4. Extrakt zur Verwendung nach einem der vorhergehenden Ansprüche, wobei der Anteil aus Sideritis scardica mindestens 50%, bevorzugt mindestens 80% beträgt.

5. Extrakt zur Verwendung nach einem der vorhergehenden Ansprüche, **dadurch gekennzeichnet, dass** die oberirdischen Pflanzenteile zum Zeitpunkt der Blüte geerntet wurden.

6. Extrakt zur Verwendung nach einem der vorhergehenden Ansprüche, **dadurch gekennzeichnet, dass** der Extrakt hergestellt wurde mit einem Auszugsmittel, ausgewählt aus der Gruppe bestehend aus Wasser, ein- und mehrwertigen Alkoholen und Ketonen, bevorzugt Alkohole oder Ketone mit 1 bis 4 C-Atomen oder deren Mischungen.

7. Extrakt zur Verwendung nach Anspruch 6, **dadurch gekennzeichnet, dass** der Extrakt hergestellt wurde mit einer Auszugsmitteltemperatur zwischen 20 und 100°C, bevorzugt bei Temperaturen 10°C unterhalb der Siedetemperatur des Auszugsmittels.

8. Extrakt zur Verwendung nach Anspruch 6, **dadurch gekennzeichnet, dass** der Extrakt mit Gefriertrocknung, Sprühtrocknung, Bandtrocknung, Vakuumtrocknung, Walzentrocknung oder einer Kombinationen dieser Verfahren getrocknet wurde.

9. Extrakt zur Verwendung nach einem der vorhergehenden Ansprüche, **dadurch gekennzeichnet, dass** der Extrakt in einer ergänzenden bilanzierten Diät oder einer pharmazeutischen Zubereitung enthalten ist.

10. Extrakt zur Verwendung nach Anspruch 9, wobei die oberirdischen Pflanzenteile oder Extrakte in Form einer Tablette, einer Kapsel, einer Kauzubereitung, einer Lutschzubereitung, einer Brausezubereitung, eines Granulates, eines Getränkes oder einer Instantzubereitung, insbesondere einer Instantteezubereitung.

11. Extrakt zur Verwendung nach einem der vorhergehenden Ansprüche in Verbindung mit physiologisch verträglichen Hilfsstoffen, vorzugsweise aus der Gruppe der Kohlehydrate, bevorzugt Stärkeabbauprodukte wie Maltodextrin, Glucosesirup, Zuckern, Zellulose und deren entsprechenden Kohlehydrat-Derivate, aus der Gruppe der Proteine wie Gummi arabicum, Kollagene sowie Kollagenhydrolysate.

12. Extrakt zur Verwendung nach einem der vorhergehenden Ansprüche in Kombination mit Nootropika, bevorzugt ausgewählt aus Acetylcholinesterase-Hemmern, insbesondere aus der Gruppe Donezepil, Galantamin, Rivastigmin, Tacrin und deren Derivate, weiter bevorzugt GABA-analoga insbesondere Piracetam und dessen Derivate, weiter bevorzugt Ergot-Derivate, insbesondere Nicergolin, Dihydroergotoxin und deren Derivate, und weiterhin bevorzugt NMDA-Antagonisten, insbesondere Memantin.

13. Verwendung von Extrakten aus oberirdischen Pflanzenteilen von Sideritis ssp. zur nicht medizinischen Steigerung kognitiver Leistungsfähigkeit, wobei es sich bei den oberirdischen Pflanzenteilen um frische oder getrocknete Teile von Pflanzen aus der Gruppe Sideritis euboa, Sideritis scardica, Sideritis raiseri oder deren Mischungen handelt.

14. Verwendung nach Anspruch 13, **dadurch gekennzeichnet, dass** der Extrakt in einem Lebensmittel oder einem Nahrungsergänzungsmittel enthalten ist.

## Claims

1. An extract from aerial plant parts of *Sideritis* ssp. for use for enhancing cognitive performance, wherein said aerial plant parts are fresh or dried parts of plants selected from the group of *Sideritis euboa, Sideritis scardica, Sideritis raiseri,* or mixtures thereof, wherein said extract has been dried, except for use in mild cognitive impairment in Alzheimer's disease, and except for non-medical use.

2. The extract for use according to claim 1, **characterized in that** said enhancement of cognitive performance is enhancement of learning performance, preferably enhancement of memory, more preferably in an existing stress situation.

3. The extract for use according to claim 2, **characterized in that** said existing stress situation is stressful situations or neurodegenerative diseases, especially exam stress in connection with new tasks or in special situations.

4. The extract for use according to any of the preceding claims, wherein the proportion from *Sideritis scardica* is at least 50%, preferably at least 80%.

5. The extract for use according to any of the preceding claims, **characterized in that** said aerial plant parts were harvested at the time of flowering.

6. The extract for use according to any of the preceding claims, **characterized in that** said extract has been prepared by means of an extractant selected from the group consisting of water, monohydric and polyhydric alcohols and ketones, preferably alcohols or ketones with 1 to 4 carbon atoms or mixtures thereof.

7. The extract for use according to claim 6, **characterized in that** said extract has been prepared with an extractant temperature of from 20 to 100 °C, preferably at temperatures 10 °C below the boiling temperature of the extractant.

8. The extract for use according to claim 6, **characterized in that** said extractant has been dried by freeze-drying, spray drying, belt conveyor drying, vacuum drying, roller drying, or a combination of such methods.

9. The extract for use according to any of the preceding claims, **characterized in that** said extract is contained in a supplementing balanced diet or in a pharmaceutical formulation.

10. The extract for use according to claim 9, wherein said aerial plant parts or extracts are in the form of a tablet, capsule, chewing formulation, sucking formulation, effervescent formulation, granules, beverage, or instant formulation, especially an instant tea formulation.

11. The extract for use according to any of the preceding claims in connection with physiologically tolerable auxiliaries, preferably from the group of carbohydrates, preferably starch degradation products, such as maltodextrin, glucose syrup, sugars, cellulose and its corresponding carbohydrate derivatives, from the group of proteins, such as gum arabic, collagens and collagen hydrolyzates.

12. The extract for use according to any of the preceding claims in combination with nootropics, preferably selected from acetylcholinesterase inhibitors, especially from the group of donezepil, galantamine, rivastigmine, tacrine and derivatives thereof, further preferably GABA analogues, especially piracetam and derivatives thereof, further preferably ergot derivatives, especially nicergoline, dihydroergotoxine and derivatives thereof, and further preferably NMDA antagonists, especially memantine.

13. Use of extracts from aerial plant parts of *Sideritis* ssp. for the non-medical enhancement of cognitive performance, wherein said aerial plant parts are fresh or dried parts of plants selected from the group of *Sideritis euboa, Sideritis scardica, Sideritis raiseri,* or mixtures thereof.

14. The use according to claim 13, **characterized in that** said extract is contained in a food or food supplement.

## Revendications

1. Extrait de parties aériennes de plantes de Sideritis ssp. pour une utilisation destinée à augmenter les performances cognitives, dans lequel les parties aériennes de plantes sont des parties fraîches ou séchées de plantes du groupe Sideritis euboa, Sideritis scardica, Sideritis raiseri ou de leurs mélanges, l'extrait étant séché, excepté pour l'utilisation en cas de trouble cognitif léger en cas de maladie d'Alzheimer et excepté pour une utilisation non médicale.

2. Extrait pour l'utilisation selon la revendication 1, **caractérisé en ce que**, en matière d'augmentation des performances cognitives, il s'agit d'une augmentation des performances d'apprentissage, de préférence d'une augmentation des performances mnésiques, de façon particulièrement préférée lors d'une situation existante de contrainte.

3. Extrait pour l'utilisation selon la revendication 2, **caractérisé en ce que**, en matière de situation existante de contrainte, il s'agit de situations de stress ou de maladies neurodégénératives, en particulier de stress d'apprentissage en liaison avec des tâches nouvelles ou dans des situations particulières.

4. Extrait pour l'utilisation selon une des revendications précédentes, dans lequel la proportion de Sideritis scardica est égale à au moins 50 %, de préférence égale à au moins 80 %.

5. Extrait pour l'utilisation selon une des revendications précédentes, **caractérisé en ce que** les parties aériennes de plantes ont été récoltées au moment de la floraison.

6. Extrait pour l'utilisation selon une des revendications précédentes, **caractérisé en ce que** l'extrait a été réalisé avec un agent d'extraction sélectionné dans le groupe composé de l'eau, d'alcools monovalents et polyvalents et de cétones, de préférence d'alcools ou de cétones comportant de 1 à 4 atomes de C ou de leurs mélanges.

7. Extrait pour l'utilisation selon la revendication 6, **caractérisé en ce que** l'extrait a été réalisé avec une température d'agent d'extraction entre 20 et 100 °C, de préférence à des températures inférieures de 10 °C à la température d'ébullition de l'agent d'extraction.

8. Extrait pour l'utilisation selon la revendication 6, caractérisé en ce l'extrait a été séché par séchage par lyophilisation, séchage par atomisation, séchage à bande, séchage sous vide, séchage sur cylindres ou par une combinaison de ces procédés.

9. Extrait pour l'utilisation selon une des revendications précédentes, **caractérisé en ce que** l'extrait est contenu dans un régime alimentaire complémentaire à apport énergétique contrôlé ou dans une préparation pharmaceutique.

10. Extrait pour l'utilisation selon la revendication 9, dans lequel les parties aériennes de plantes ou les extraits se présentent sous forme de comprimé, de capsule, de préparation à mâcher, de préparation à sucer, de préparation effervescente, de granulé, de boisson ou de préparation instantanée, en particulier de préparation de thé instantané.

11. Extrait pour l'utilisation selon une des revendications précédentes en association avec des adjuvants physiologiquement compatibles, de préférence appartenant au groupe des hydrates de carbone, de préférence des produits de dégradation de l'amidon tels que la maltodextrine, le sirop de glucose, des sucres, la cellulose et leurs dérivés d'hydrates de carbone correspondants, appartenant au groupe des protéines tels que la gomme arabique, des collagènes ainsi que des hydrolysats de collagène.

12. Extrait pour l'utilisation selon une des revendications précédentes en combinaison avec des nootropes, de préférence sélectionnés parmi des inhibiteurs de l'acétylcholinestérase, en particulier parmi le groupe donépézil, galantamine, rivastigmine, tacrine et leurs dérivés, plus préférablement des analogues du GABA en particulier le piracétame et ses dérivés, plus préférablement des dérivés de l'ergot, en particulier la nicergoline, la dihydroergotoxine et leurs dérivés et encore plus préférablement des antagonistes NMDA, en particulier la mémantine.

13. Utilisation d'extraits de parties aériennes de plantes de Sineritis ssp. pour l'augmentation non médicale de performances cognitives, dans lequel les parties aériennes de plantes sont des parties fraîches ou séchées de plantes du groupe Sideritis euboa, Sideritis scardica, Sideritis raiseri ou de leurs mélanges.

14. Utilisation selon la revendication 13, **caractérisée en ce que** l'extrait est contenu dans un aliment ou dans un complément alimentaire.
